# EUROPEAN PATENT SPECIFICATION

(11) **EP 1 765 279 B1**
(45) Date of publication and mention of the grant of the patent: **10.11.2010**
(21) Application number: 05752575.0
(22) Date of filing: 04.04.2005
(51) Int. Cl.: A61K 8/18, A61K 8/99

(54) **METHOD FOR THE PREPARATION OF COSMETIC EMULSIONS**
VERFAHREN ZUR HERSTELLUNG VON KOSMETISCHEN EMULSIONEN
MÉTHODE DE PRÉPARATION D'ÉMULSIONS POUR LA COSMÉTIQUE

(30) Priority: 05.04.2004 US 559832 P; 08.07.2004 EP 04016057
(43) Date of publication of application: 28.03.2007
(73) Proprietor: SemBioSys Genetics Inc., Calgary, AB T1Y 7L3 (CA)
(72) Inventor: GUTH, Jacob, Upper Blach Eddy, PA 18972 (US); LENTNER, Vickie, Washington, NJ 07882 (US)
(74) Representative: Williams, Aylsa
(86) International application number: PCT/EP2005/003501
(87) International publication number: WO 2005/097059

(56) References cited:
- EP-A- 0 998 897
- EP-A- 1 250 916
- WO-A-03/047534
- DE-A- 3 304 897
- RU-C- 2 126 247
- US-B1- 6 183 762
- US-B2- 6 582 710

## Description

### FIELD OF THE INVENTION

This invention relates to a method for the preparation of oil-in-water emulsions suitable for cosmetic or topical dermatological products.

### BACKGROUND OF THE INVENTION

Oil-in-water (O/W) emulsions typically contain between 5 and 40% by weight of oil and/or oil soluble (hydrophobic) ingredients and between 60 and 95% by weight of water and/or water soluble (hydrophilic) ingredients.

O/W emulsions are normally prepared by combining the oil phase and the water phase together at high temperature (80-85 °C) and with vigorous agitation in the presence of an emulsifier until the oil droplets are completely dispersed within the water. The emulsifying agent is generally part of the oil phase, and is present at a level of between 10 and 30% by weight of the total oil phase. The resulting emulsion must then be continuously agitated during the cooling process in order to prevent separation (of the emulsion) into the original two phases. Typically agitation is required until the emulsion has cooled to a temperature of 40 °C or below. The process of heating the oil and water phases in order to achieve the emulsion is very time and energy consuming as is the cooling down phase. Moreover, in products for topical application the presence of the emulsifier in the finished emulsion presents a number of drawbacks:
1) The presence of the emulsifier reduces the water repellency of the formulation once it has been applied to the skin. This is particularly important for sunscreen applications and color cosmetics.
2) In skin care products, the presence of the emulsifier increases the irritancy potential of the formulation, in that it has a delipidizing effect on the skin.

Vegetable oil bodies (oleosomes) or oil microspheres are discrete spheres which occur naturally in the seeds of oilseed crops. They consist of an outer coat of phospholipids and proteins (oleosins) and an internal liquid, semi-solid, or low melting solid collection of the triglycerides associated with the individual plant seed. For example, safflower oil microspheres (oleosomes) would contain the triglycerides with the fatty acid content normally associated with that plant seed, i.e., 6.5% palmitic, 2.5% stearic, 11.5% oleic, 79% linoleic, and 0.5% linolenic acids. Size and properties of these microspheres are dependent on the seed type, but for safflower seeds the microspheres are typically 3 µm in diameter and can be obtained as a 70-75 wt.% emulsion in water.

The preparation and several applications of oil bodies (oleosomes) are described in a number of patents assigned to the Canadian company Sembiosys Genetics Inc., e.g. U.S. Patent No. 6,146,645. Three of these patents are discussed in the following.

Deckers et al., U.S. Patent No. 6,183,762, disclose the preparation of cosmetic compositions for various applications. However, in each one of the applications described, the oil and water phases of the emulsion are heated to temperatures of 70 °C or higher whereupon they are added together with agitation. After the emulsion has been prepared and cooling has taken place (to 40°C or lower ) then the oil body (microsphere) emulsion is added.

In U.S. Patent No. 6,582,710, the same inventors claim the use of vegetable oil microspheres (oleosomes) in cosmetic applications. Again all of the formulations are prepared in the traditional way, i.e. through the process of heating the oil and water phases independently, combining them together with agitation, and then cooling to 40°C before addition of the oleosomes.

Another patent by the same inventors, U.S. Patent No. 6,599,513, describes the use of oil bodies to achieve finished formulations applicable to cosmetics. Again all of the Examples comprise a heating step.

### SUMMARY OF THE INVENTION

We have found that we can prepare stable, cosmetically acceptable emulsions simply by adding the requisite water or oil soluble ingredients directly to the dispersion of washed oil bodies (oleosomes) with stirring. No lengthy heating period is required and obviously no cooling period is needed either; the process comprises no heating step at all. The advantages of such an approach in terms of energy savings, preparation time, and in increased product throughput should be obvious.

### DETAILED DESCRIPTION OF THE INVENTION

As hereinbefore mentioned, the cosmetic or topical dermatological emulsions are prepared according to the present invention by adding the cosmetically or dermatologically active ingredients and, optionally, water to a dispersion of washed oil bodies (oleosomes) under stirring without any heating, at a temperature never exceeding about 40°C, to obtain an oil-in-water emulsion containing at least 60% by weight of water. The order of addition is not critical and the ingredients may be added one at a time or in premixed form, e.g. water-soluble components as aqueous solution or mutually miscible liquid ingredients as a mixture. Additional oil soluble ingredients (e.g. UV chromophores, vitamins, etc.) as well as other adjuvants and additives can also be added to the dispersion of oil bodies. The stirring can be effected by essentially any sort of stirrer or impeller operated at appropriate speed.

The cosmetic or topical dermatological products that can be prepared by the method of the present invention are essentially all products comprising oil-in-water emulsions in lotion, cream or paste form, including, but not limited to, moisturizing preparations, ointments, skin reparatives, sunscreens, skin lighteners, and decorative cosmetics such as color foundation creams, eye shadow creams and tanner creams.

Accordingly, they may comprise ingredients designed to exfoliate, repair, color, lighten, moisturize, smooth, condition, protect, prevent the moisture loss of, reverse the damage of, and clean and rejuvenate the skin and hair.

Such ingredients may include but are not limited to alcohols, esters, fatty acids, hydrocarbons, silicones, amines, enzymes, salts of amines and salts of fatty acids.

Other ingredients which could be added would include thickeners, barrier agents, UV chromophores, emollients, emulsifiers, conditioners, vitamins, etc.

Preferably, the emulsion is a cosmetic product is selected from the group consisting of moisturizers, ointments, cleansers, makeup removers, toners, night treatments, skin reparatives, sunscreens, skin lighteners, color foundations, eye shadows and tanners.

In one preferred embodiment, the product is a lotion.

In another preferred embodiment, the product is a cream.

The vegetable oil bodies (oleosomes) can be obtained from any plant cells, including cells from pollens, spores, seeds and vegetative plant organs in which oil bodies or oil body-like organelles are present. Preferably, the vegetable oil bodies are obtained from plant seeds or fruits, more preferably from the group of plant species consisting of rapeseed, soybean, sunflower, oil palm, cottonseed, peanut, walnut, coconut, castor, safflower, mustard, coriander, squash, linseed, brazil nut, jojoba, avocado and maize. Most preferably, the vegetable oil bodies are obtained from safflower seeds.

The preparation of the vegetable oil bodies (oleosomes) is disclosed in several patents, for example, U.S. Pat. No. 6,146,645. The preparation process comprises at least one washing step to accomplish removal of contaminating seed material, which is essential for obtaining a dispersion having properties suitable for being used in cosmetics. Furthermore, said removal of contaminating material reduces the risk of allergic reactions when the cosmetic preparation is applied to the skin.

The method of the present invention requires no additional emollients, emulsifiers etc. which constitutes a substantial advantage over the methods and productions of prior art. In the method of the present invention, no additional emulsifier is used.

The oleosomes are preferably used in an amount of from 2 wt% to 15 wt.%, more preferably 5 wt% to 12 wt%, of a 70-75 wt% emulsion in water, based on the total weight of the cosmetic or topical dermatological product.

The cosmetic or topical dermatological product produced according to the method of the present invention are remarkably stable and do not show any phase separation even after several months at elevated temperatures of e.g. 45 °C.

The following non-limiting examples are illustrative of the present invention:

### EXAMPLES

### General procedure:

All preparations have been carried out using a 2 L beaker and a Heidolph^{®} RZR50 stirrer equipped with an anchor-type impeller of 10 cm diameter The stirring speed is given in min⁻¹ (1 min⁻¹ = 1 revolution per minute ≡ 1 rpm). The batch size was 1 kg in each example.

### Materials:

All materials used in the examples are commercially available, in particular.

Stearic acid, octyl methoxycinnamate, paraffin oil, cetyl alcohol, urea, vitamin A, vitamin E, glycerol, arbutin, polysorbate 80 and hydroxyethylcellulose are standard chemicals obtainable from several suppliers. Decaglycerol L DE (Natrulon^{®} H 10), L-carnitine, Geogard^{®} 361, Lonzest^{®} 143S (myristyl propionate), sorbitan monooleate and Aldo^{®} MCT (medium chain triglycerides) are supplied by Lonza Inc., Allendale, NJ, USA. Novemer^{®} EC-1 is available from Noveon Inc., Cleveland, OH, USA. Almond fragrance 901A79 is available from Flavor & Fragrance Specialties Inc., Mahwah, NJ, USA. Safflower (*Carthamus tinctorius* L.) oil microspheres (oleosomes) are available from Sembiosys Genetics Inc., Calgary, Canada. Kelzan^{®}-T is available from CP Kelco, Wilmington, DE, USA. The pigments used in examples 8 to 13, i.e., green color pigment, red cosmetic iron oxide, brown iron oxide and titanium dioxide are supplied by Whittaker, Clark & Daniels Inc., South Plainfield, NJ, USA. Papain is available from Enzybel S.A., Villers-le-Bouillet, Belgium and bromelain from Marcor Development Corp., Carlstadt, NJ, USA. *Rumex occidentalis* extract is obtainable from Atrium Biotechnologies Inc., Fairfield, NJ, USA.

An example of a typical sunscreen formulation prepared in the traditional way is shown below in Comparative Example 1. The process for preparation of a similar sunscreen via a "cold process" approach is shown in Example 1. Examples 2 to 15 relate to various cosmetic formulations.

### Comparative Example 1

**Sunscreen (SPF≅ 13)**

| **Ingredients** | **% Weight** |
|---|---|
| Stearic Acid | 6.00 |
| Octyl Methoxycinnamate | 8.00 |
| Paraffin Oil | 3.00 |
| Cetyl Alcohol | 2.00 |
| Myristyl Propionate (Lonzest^{®} 143S) | 3.00 |
| Medium Chain Triglycerides (Aldo^{®} MCT) | 3.00 |
| Sorbitan Monooleate | 5.05 |
| Polysorbate 80 | 3.95 |
| Water | 65.60 |
| Geogard^{®} 361 (Preservative) | 0.40 |
| Novemer^{®} EC-1 | 1.00 |
| Almond Fragrance # 901A79 | q.s. |

| | |
|---|---|
| SPF = sun protection factor, q.s. = quantum sufficit, "≅" = approximately | |

### Procedure

Stearic acid, octyl methoxycinnamate, paraffin oil, cetyl alcohol, myristyl propionate, medium chain triglycerides, sorbitan monooleate and polysorbate 80 are mixed (phase 1) and heated to 80-85 °C with stirring to avoid charring and overheating. Water (phase 2) is heated to 80-85 °C. Phase 1 is added to phase 2 with vigorous stirring over a 30 minute time period. The temperature is maintained at 80 °C for an additional 30 min while agitation continues. The emulsion is then cooled while stirring vigorously until a temperature of 40 °C or less is achieved, whereupon the preservative, Novemer^{®} and the fragrance is added. Total process time is approximately 4.5 hours.

### Example 1

**Sunscreen (SPF≅ 15)**

| **Ingredients** | **% Weight** |
|---|---|
| Octyl Methoxycinnamate | 8.00 |
| Water | 76.60 |
| Safflower Oleosomes (70%) | 8.00 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes and mix at 500 min⁻¹ for 10 min. Add the decaglycerol to the mixture with stirring for 2 min at 500 min⁻¹. Add the methoxycinnamate and the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 10,240 mPa·s The formulation passed stability testing at 45 °C for two months.

Thus the formulation of Example 1 in which safflower oleosomes were substituted for the diluents, emollients and emulsifiers that were used in the Comparative Example 1 was prepared with no heating, no cooling, in much less time, and with far fewer ingredients.

### Example 2

**Emulsifier Free Sunscreen**

| **Ingredients** | **% Weight** |
|---|---|
| Octyl Methoxycinnamate | 8.00 |
| Water | 76.60 |
| Safflower Oleosomes (70%) | 8.00 |
| Decaglycerol L DE (Natrulon^{®} H 10) | 5.00 |
| Hydroxyethylcellulose (Natrosol^{®}) | 2.00 |
| Geogard^{®} 361 (Preservative) | 0.40 |
| Almond Fragrance # 901A79 | q.s. |

### Procedure

Add the water to the safflower oleosomes and mix at 500 min⁻¹ for 5 min. Add the decaglycerol to the mixture with stirring for 2 min at 500 min⁻¹. Add the methoxycinnamate and the preservative to the mixture and stir at 500 min⁻¹ for 8 min. Add Natrosol^{®} (presolubilized) and stir at 500 min⁻¹ until the mixture begins to thicken and then increase the stirrer speed to 600 min⁻¹ and stir for an additional 15 min. Total preparation time is ∼30 min, viscosity 22,800 mPa·s. The formulation passed stability testing at 40 °C for two months.

Many other cosmetic or topical dermatological products can be prepared in stable form via the cold process route, for example reparative creams/lotions, moisturizing creams/lotions, lightener creams, ointment creams, exfoliating creams, eye shadow creams, color foundation creams or tanner creams (Examples 3-15).

### Example 3

**Reparative Cream**

| **Ingredients** | **% Weight** |
|---|---|
| Octyl Methoxycinnamate | 8.00 |
| Water | 78.60 |
| Safflower Oleosomes (70%) | 7.00 |
| L-Carnitine | 1.00 |
| Decaglycerol L DE (Natrulon^{®} H 10) | 3.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes along with the L-carnitine and mix at 500 min⁻¹ for 10 min. Add the decaglycerol to the mixture with stirring for 2 min at 500 min⁻¹. Add the methoxycinnamate and the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 17,800 mPa·s. The formulation passed stability testing at 45 °C for two months.

### Example 4

**Moisturizing Cream**

| **Ingredients** | **% Weight** |
|---|---|
| Octyl Methoxycinnamate | 8.00 |
| Water | 69.60 |
| Safflower Oleosomes (70%) | 10.00 |
| Urea | 5.00 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with urea and mix at 500 min⁻¹ for 10 min. Add the decaglycerol to the mixture with stirring for 2 min at 500 min⁻¹. Add the methoxycinnamate and the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 17,800 mPa·s. The formulation passed stability testing at 45 °C for two months.

### Example 5

**Lightener Cream**

| **Ingredients** | **% Weight** |
|---|---|
| Water | 84.00 |
| Safflower Oleosomes (70%) | 7.00 |
| Arbutin | 1.00 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with arbutin and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 13,220 mPa·s. The formulation passed stability testing at 45 °C for two months.

### Example 6

**Ointment Cream with Vitamin A and E**

| **Ingredients** | **% Weight** |
|---|---|
| Water | 84.00 |
| Safflower Oleosomes (70%) | 10.00 |
| Vitamin A | 0.5 |
| Vitamin E | 0.5 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 90 1 A79 | q.s. |

### Procedure

Add the water to the safflower oleosomes with vitamin A and E and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 12,320 mPa·s. The formulation passed stability testing at 45 °C for two months.

### Example 7

**Botanical Exfoliating Cream**

| **Ingredients** | **% Weight** |
|---|---|
| Water | 72.60 |
| Safflower Oleosomes (70%) | 10.00 |
| Enzyme concentrate¹⁾ | 2.00 |
| Decaglycerol L DE (Natrulon^{®} H 10) | 5.00 |
| Novemer^{®} EC-1 | 2.00 |
| Geogard^{®} 361 (Preservative) | 0.40 |
| Octyl Methoxycinnamate | 8.00 |
| Almond Fragrance # 901A79 | q.s. |

| | |
|---|---|
| ¹⁾ 18.9 mg papain (62000 PU) + 4.5 mg bromelain (3300 GDU) per 2 g solution (solvent: 50/50 mixture of decaglycerol and water) | |

### Procedure

Add the water to the safflower oleosomes with papain and bromelain and mix at 500 min⁻¹ for 10 min. Add the decaglycerol to the mixture with stirring for 2 min at 500 min⁻¹. Add the methoxycinnamate and the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 17,310 mPa·s. The formulation passed stability testing at 45 °C for two months.

### Example 8

**Eye Shadow Cream**

| **Ingredients** | **% Weight** |
|---|---|
| Water | 80.30 |
| Safflower Oleosomes (70%) | 12.00 |
| Green Color Pigment A2-2502 | 0.30 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with green color pigment and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC⁻¹ and stir at 400 min⁻¹ until the mixture begins to thicken and then increase the stirrer speed to 600 min⁻¹ and stir for an additional 3 min. Total preparation time is ∼23 min, viscosity 17,820 mPa·s. The formulation passed stability testing at 45 °C for two months.

### Example 9

**Color Foundation Cream**

| **Ingredients** | **% Weight** |
|---|---|
| Water | 80.10 |
| Safflower Oleosomes (70%) | 12.00 |
| Red Cosmetic Iron Oxide | 0.50 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with red iron oxide color pigment and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 17,200 mPa·s. The formulation passed stability testing at 45 °C for two months.

### Example 10

**Color Foundation/Tanner Cream**

| **Ingredients** | **% Weight** |
|---|---|
| Water | 80.10 |
| Safflower Oleosomes (70%) | 12.00 |
| Brown Iron Oxide # E 172 | 0.50 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with brown iron oxide color pigment and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 16,200 mPa·s. The formulation passed stability testing at 45 °C for two months.

### Example 11

**Color Blush/Eye Shadow Cream**

| **Ingredients** | **% Weight** |
|---|---|
| Water | 82.35 |
| Safflower Oleosomes (70%) | 10.00 |
| Red Cosmetic Iron Oxide | 0.25 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with red cosmetic iron oxide color pigment and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 16,890 mPa·s. The formulation passed stability testing at 45 °C for two months.

### Example 12

**Color Blush/Eye Shadow Cream**

| **Ingredients** | **% Weight** |
|---|---|
| Water | 77.60 |
| Safflower Oleosomes (70%) | 10.00 |
| Red Cosmetic Iron Oxide | 0.25 |
| Decaglycerol L DE (Natrulon^{®} H 10) | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with red cosmetic iron oxide color pigment and mix at 500 min⁻¹ for 10 min. Add the decaglycerol to the mixture with stirring for 2 min at 500 min⁻¹. Add the preservative to the mixture and stir at 500 min⁻¹ for 8 min. Add the Novemer^{®} EC-1 and stir at 400 min⁻¹ until the mixture begins to thicken and then increase the stirrer speed to 600 min⁻¹ and stir for an additional 3 min. Total preparation time is ∼23 min, viscosity 16,890 mPa·s. The formulation passed stability testing at 45 °C for two months.

### Example 13

**Emulsifier Free Lightener/Exfoliating Cream**

| **Ingredients** | **% Weight** |
|---|---|
| Water | 77.60 |
| Safflower Oleosomes (70%) | 10.00 |
| Urea | 1.00 |
| Glycerol | 2.00 |
| *Rumex occidentalis* Extract | 1.00 |
| Kelzan^{®}-T | 3.00 |
| Titanium Dioxide (Whittaker, Clark & Daniels # 3328) | 5.00 |
| Geogard^{®} 361 (Preservative) | 0.40 |
| Almond Fragrance # 901A79 | q.s. |

### Procedure

Add the water to the safflower oleosomes with urea and mix at 500 min⁻¹ for 10 min. Add the glycerol and titanium dioxide and *Rumex occidentalis* extract with stirring for 2 min at 500 min⁻¹. Add the preservative to the mixture and stir at 500 min⁻¹ for 8 min. Add the Kelzan^{®}-T and stir at 400 min⁻¹ until the mixture begins to thicken and then increase the stirrer speed to 600 min⁻¹ and stir for an additional 3 min. Total preparation time is -23 min, viscosity 14,500 mPa·s. The formulation passed stability testing at 45 °C for two months.

### Example 14

**Reparative Cream For Dry Skin**

| **Ingredients** | **% Weight** |
|---|---|
| Octyl Methoxycinnamate | 8.00 |
| Water | 73.60 |
| Safflower Oleosomes (70%) | 10.00 |
| L-Carnitine | 1.00 |
| Decaglyceryl decaoleate | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes along with the L-carnitine and mix at 500 min⁻¹ for 10 min. Add the decaglycerol decaoleate to the methoxycinnamate and the preservative to the mixture and stir at 500 min⁻¹ for 8 min. Add the Novemer^{®} EC-1 and stir at 400 min⁻¹ (5 min at 500 min⁻¹) until the mixture begins to thicken and then increase the stirrer speed to 600 min⁻¹ and stir for an additional 3 min. Total preparation time is ∼26 min, viscosity 17,800 mPa·s. The formulation passed stability testing at 45 °C for two months.

### Example 15

**Moisturizing Cream For Dry Skin**

| **Ingredients** | **% Weight** |
|---|---|
| Octyl Methoxycinnamate | 8.00 |
| Water | 69.60 |
| Safflower Oleosomes (70%) | 10.00 |
| Urea | 5.00 |
| Decaglyceryl decaoleate | 5.00 |
| Novemer^{®} EC-1 | 2.00 |
| Geogard^{®} 361 (Preservative) | 0.40 |
| Almond Fragrance # 901A79 | q.s. |

### Procedure

Add the water to the safflower oleosomes with urea and mix at 500 min⁻¹ for 10 min. Add the decaglyceryl decaoleate to the methoxycinnamate and the preservative to the mixture and stir at 500 min⁻¹ for 10 min. Add the Novemer^{®} EC-1 and stir at 400 min⁻¹ (2 min) until the mixture begins to thicken and then increase the stirrer speed to 600 min⁻¹ and stir for an additional 3 min. Total preparation time is ∼25 min, viscosity 17,800 mPa·s. The formulation passed stability testing at 45 °C for two months.

## Claims

1. A method for preparing a cosmetic or topical dermatological product comprising an oil-in-water emulsion comprising at least 60% by weight of water, the method comprising the step of mixing at least one cosmetically or dermatologically active ingredient with an aqueous emulsion of washed vegetable oil bodies, wherein no additional emulsifier is used and wherein the temperature of said ingredients and said emulsion does never exceed about 40°C.

2. The method of claim 1, wherein the product is a cosmetic product is selected from the group consisting of moisturizers, ointments, cleansers, makeup removers, toners, night treatments, skin reparatives, sunscreens, skin lighteners, color foundations, eye shadows and tanners.

3. The method of claim 1 or 2, wherein the product is a lotion.

4. The method of claim 1 or 2, wherein the product is a cream.

5. The method of any of claim 1 to 4, wherein the vegetable oil bodies are selected from the group consisting of oil bodies obtained from rapeseed, soybean, sunflower, oil palm, cottonseed, peanut, walnut, coconut, castor, safflower, mustard, coriander, squash, linseed, brazil nut, jojoba, avocado and maize.

6. The method of claim 5, wherein the vegetable oil bodies are obtained from safflower.

7. The method of any of claims 1 to 6, wherein the vegetable oil bodies are used in an amount of from 2 wt.% to 15 wt.% of a 70-75 wt.% aqueous emulsion, based on the total weight of the cosmetic or topical dermatological product.

8. A cosmetic or topical dermatological product comprising an oil-in-water emulsion comprising at least 60% by weight of water, wherein the product is emulsifier free and is made by a method comprising the step of mixing at least one cosmetically or dermatologically active ingredient with an aqueous emulsion of washed vegetable oil bodies.

9. The product of claim 8, wherein the product is a cosmetic product is selected from the group consisting of moisturizers, ointments, cleansers, makeup removers, toners, night treatments, skin reparatives, sunscreens, skin lighteners, color foundations, eye shadows and tanners.

10. The product of claim 8 or 9, wherein the product is a lotion.

11. The product of claim 8 or 9, wherein the product is a cream.

12. The product of any of claims 8 to 11, wherein the vegetable oil bodies are selected from the group consisting of oil bodies obtained from rapeseed, soybean, sunflower, oil palm, cottonseed, peanut, walnut, coconut, castor, safflower, mustard, coriander, squash, linseed, brazil nut, jojoba, avocado and maize.

13. The product of claim 12, wherein the vegetable oil bodies are obtained from safflower.

14. The product of any of claims 8 to 13, wherein the temperature of said ingredients and said emulsion does never exceed about 40°C.

15. The product of any of claims 8 to 14, wherein the vegetable oil bodies are used in an amount of from 2 wt.% to 15 wt.% of a 70-75 wt.% aqueous emulsion, based on the total weight of the cosmetic or topical dermatological product.

## Patentansprüche

1. Verfahren zur Herstellung eines kosmetischen oder topischen dermatologischen Produkts mit einer Öl-in-Wasser-Emulsion, umfassend wenigstens 60 Gew.-% Wasser, wobei das Verfahren die Stufe des Mischens von wenigstens einem kosmetisch oder dermatologisch aktiven Bestandteil mit einer wässrigen Emulsion von gewaschenen Pflanzenölkörpern umfasst, wobei kein zusätzlicher Emulgator verwendet wird, und wobei die Temperatur der Bestandteile und der Emulsion etwa 40°C niemals überschreitet.

2. Verfahren nach Anspruch 1, wobei das Produkt ein kosmetisches Produkt ist, das ausgewählt ist aus der Gruppe, bestehend aus Feuchtigkeitsmitteln, Salben, Reinigungsmitteln, Makeup-Entfernern, Gesichtsreinigungsmitteln, Behandlungsmitteln für die Nacht, Hautreparaturmitteln, Sonnenschutzmitteln, Hautaufhellern, Farbgrundlagen, Lidschatten und Bräunungsmitteln.

3. Verfahren nach Anspruch 1 oder 2, wobei das Produkt eine Lotion ist.

4. Verfahren nach Anspruch 1 oder 2, wobei das Produkt eine Creme ist.

5. Verfahren nach einem der Ansprüche 1 bis 4, wobei die pflanzlichen Ölkörper ausgewählt sind aus der Gruppe, bestehend aus Ölkörpern, die gewonnen sind aus Rapssamen, Sojabohnen, Sonnenblume, Ölpalme, Baumwollsamen, Erdnuss, Wallnuss, Kokosnuss, Rizinus, Saflor, Senf, Koriander, Kürbis, Leinsamen, Paranuss, Jojoba, Avocado und Mais.

6. Verfahren nach Anspruch 5, wobei die Pflanzenölkörper aus Saflor gewonnen sind.

7. Verfahren nach einem der Ansprüche 1 bis 6, wobei die Pflanzenölkörper in einer Menge von 2 Gew.-% bis 15 Gew.-% von einer 70-75 Gew.-%-Wasseremulsion, bezogen auf das Gesamtgewicht des kosmetischen oder topischen dermatologischen Produkts, verwendet werden.

8. Kosmetisches oder topisches dermatologisches Produkt, umfassend eine Öl-in-Wasser-Emulsion, umfassend wenigstens 60 Gew.-% Wasser, wobei das Produkt emulgatorfrei ist und nach einem Verfahren hergestellt ist, welches die Stufe des Mischens von wenigstens einem kosmetisch oder dermatologisch aktiven Bestandteil mit einer wässrigen Emulsion von gewaschenen Pflanzenölkörpern umfasst.

9. Produkt nach Anspruch 8, wobei das Produkt ein kosmetisches Produkt ist, welches ausgewählt ist aus der Gruppe bestehend aus Feuchtigkeitsmitteln, Salben, Reinigungsmitteln, Makeup-Entfernern, Gesichtsreinigungsmitteln, Behandlungsmitteln für die Nacht, Hautreparaturmitteln, Sonnenschutzmitteln, Hautaufhellern, Farbgrundlagen, Lidschatten und Bräunungsmitteln.

10. Produkt nach Anspruch 8 oder 9, wobei das Produkt eine Lotion ist.

11. Produkt nach Anspruch 8 oder 9, wobei das Produkt eine Creme ist.

12. Produkt nach einem der Ansprüche 8 bis 11, wobei die Pfilanzenölkörper ausgewhält sind aus der Gruppe, bestehend aus Ölkörpern, die gewonnen sind aus Rapssamen, Sojabohnen, Sonnenblume, Ölpalme, Baumwollsamen, Erdnuss, Wallnuss, Kokosnuss, Rizinus, Saflor, Senf, Koriander, Kürbis, Leinsamen, Paranuss, Jojoba, Avocado und Mais.

13. Produkt nach Anspruch 12, wobei die Pflanzenölkörper aus Saflor gewonnen sind.

14. Produkt nach einem der Ansprüche 8 bis 13, wobei die Temperatur der Bestandteile und der Emulsion etwa 40°C nie übersteigt.

15. Produkt nach einem der Ansprüche 8 bis 14, wobei die Pflanzenölkörper in einer Menge von 2 Gew.-% bis 15 Gew.-% einer 70-75 Gew.-%-Wasseremulsion, bezogen auf das Gesamtgewicht des kosmetischen oder topischen dermatologischen Produkts, verwendet werden.

## Revendications

1. Procédé de préparation d'un produit cosmétique ou dermatologique topique comprenant une émulsion huile dans eau comprenant au moins 60 % en poids d'eau, le procédé comprenant l'étape de mélange d'au moins un principe actif sur le plan cosmétique ou dermatologique avec une émulsion aqueuse de corps huileux lavés d'origine végétale, dans lequel aucun émulsifiant supplémentaire n'est utilisé et dans lequel la température desdits principes et de ladite émulsion ne dépasse jamais environ 40 °C.

2. Procédé selon la revendication 1, dans lequel le produit est un produit cosmétique qui est choisi dans le groupe constitué des hydratants, pommades, nettoyants, démaquillants, toniques, traitements de nuit, réparateurs cutanés, crèmes solaires, éclaircissants cutanés, fonds de teint colorés, fards à paupières et bronzants.

3. Procédé selon la revendication 1 ou 2, dans lequel le produit est une lotion.

4. Procédé selon la revendication 1 ou 2, dans lequel le produit est une crème.

5. Procédé selon l'une quelconque des revendications 1 à 4, dans lequel les corps huileux d'origine végétale sont choisis dans le groupe constitué des corps huileux obtenus à partir de la graine de colza, de la fève de soja, du tournesol, du palmier à huile, de la graine de coton, de la cacahouète, de la noix, de la noix de coco, du ricin, du carthame, de la moutarde, de la coriandre, de la courge, de la graine de lin, de la noix du Brésil, du jojoba, de l'avocat et du maïs.

6. Procédé selon la revendication 5, dans lequel les corps huileux d'origine végétale sont obtenus à partir du carthame.

7. Procédé selon l'une quelconque des revendications 1 à 6, dans lequel les corps huileux d'origine végétale sont utilisés en une quantité de 2 % en poids à 15 % en poids d'une émulsion aqueuse de 70 % à 75 % en poids, sur la base du poids total du produit cosmétique ou dermatologique topique.

8. Produit cosmétique ou dermatologique topique comprenant une émulsion huile dans eau comprenant au moins 60 % en poids d'eau, dans lequel le produit est exempt d'émulsifiant et est préparé par un procédé comprenant l'étape de mélange d'au moins un principe actif sur le plan cosmétique ou dermatologique avec une émulsion aqueuse de corps huileux lavés d'origine végétale.

9. Produit selon la revendication 8, dans lequel le produit est un produit cosmétique qui est choisi dans le groupe constitué des hydratants, pommades, nettoyants, démaquillants, toniques, traitements de nuit, réparateurs cutanés, crèmes solaires, éclaircissants cutanés, fonds de teint colorés, fards à paupières et bronzants.

10. Produit selon la revendication 8 ou 9, dans lequel le produit est une lotion.

11. Produit selon la revendication 8 ou 9, dans lequel le produit est une crème.

12. Produit selon l'une quelconque des revendications 8 à 11, dans lequel les corps huileux d'origine végétale sont choisis dans le groupe constitué des corps huileux obtenus à partir de la graine de colza, de la fève de soja, du tournesol, du palmier à huile, de la graine de coton, de la cacahouète, de la noix, de la noix de coco, du ricin, du carthame, de la moutarde, de la coriandre, de la courge, de la graine de lin, de la noix du Brésil, du jojoba, de l'avocat et du maïs.

13. Produit selon la revendication 12, dans lequel les corps huileux d'origine végétale sont obtenus à partir du carthame.

14. Produit selon l'une quelconque des revendications 8 à 13, dans lequel la température desdits principes et de ladite émulsion ne dépasse jamais environ 40 °C.

15. Produit selon l'une quelconque des revendications 8 à 14, dans lequel les corps huileux d'origine végétale sont utilisés en une quantité de 2 % en poids à 15 % en poids d'une émulsion aqueuse de 70 % à 75 % en poids, sur la base du poids total du produit cosmétique ou dermatologique topique.
